# EUROPEAN PATENT APPLICATION

(11) **EP 2 457 916 A1**
(43) Date of publication of application: **30.05.2012**
(21) Application number: 10014657.0
(22) Date of filing: 16.11.2010
(51) Int. Cl.: C07D 498/04, G01N 33/533

(54) **Compound for the covalent attachment of the chemiluminescent probe N-(4-Aminobutyl)-N-ethylisoluminol (ABEI) to target molecules and uses thereof**

(71) Applicant: DiaSorin S.p.A., 13040 Saluggia VC (IT)
(72) Inventor: Peri, Francesco, 13040 Saluggia (VC) (IT); Palmioli, Alessandro, 13040 Saluggia (VC) (IT); Olson, Greg, Lakeland MN Minesota 55043 (US); Cummings, Steven E., Spring Valley WI Wisconsin 54767 (US)
(74) Representative: Capasso, Olga

(57) **Abstract**

The invention relates to a new molecule/reagent family, *N*-(4-Aminobutyl)-*N*-ethylisoluminol anhydroglutarate (ABEI anhydroglutarate), for the covalent attachment of the chemiluminescent label *N*-(4-Aminobutyl)-*N*-ethylisoluminol (ABEI) to nucleophilic group containing molecules, as peptides and proteins, including antibodies, for the generation of labeled probes to be used in chemiluminescence-based immunoassays.

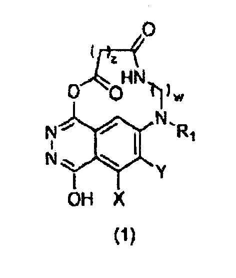

## Description

### Field of the invention

The invention relates to a new molecule/reagent family, *N-*(4-Aminobutyl)-*N* ethylisoluminol anhydroglutarate (ABEI anhydroglutarate), for the covalent attachment of the chemiluminescent label *N*-(4-Aminobutyl)-*N*-ethylisoluminol (ABEI) to nucleophilic group containing molecules, as peptides and proteins, including antibodies, for the generation of labeled probes to be used in chemiluminescence-based immunoassays. The improved chemical properties, including stability and reactivity, of the reagent have been investigated and compared with the equivalent reagent currently used for ABEI labeling, the ABEI *N*-hydroxysuccinimide (NHS) active ester.

### Background

Assays that utilize luminescence emissions are highly sensitive, quantitative, reproducible, less hazardous and less costly than radioisotope-based methodologies, They also have extremely broad applications in fields such as food testing, forensic science, environmental contaminant screening, microbiology and biomedical analysis [Whitehead, T.P., Kricka L.J., Carter T.J.N., Thorpe G.H.G. Analytical luminescence: its potential in the clinical laboratory, Clin. Chem. 1979, 25/9, 1531-1546; Case, J.F., Herring, P.J., Robison, B.H., Haddock, S.H.D., Stanley, P.E., Kricka, L.J. Bioluminescence and Chemiluminescence, World Scientific, Singapore, 2001].

Luminescence is the emission of light from excited electronic states of atoms or molecules and generally refers to all forms of light emission, except incandescence, and may include photoluminescence, chemiluminescence and electroluminescence. In chemiluminescence, the excited electronic state is created by the transfer of chemical energy. [Van Dyke, K., Van Dyke, R. Luminescence Immunoassay and Molecular Applications, CRC Press, Boca Raton, 1990].

Chemiluminometric-based assays have a number of advantages over photoluminometric-based assays including simplicity of detection and sensitivity. Unlike fluorescence which requires an incident light source for its induction, chemiluminescence is by definition its own light source. Thus, luminometric assays only require instrumentation that detects light. Such simple requirements make chemiluminescent assays robust and quick which is advantageous in maximizing through-put on automated immunoassay analyzers.

The incorporation of a chemiluminescent signal into an assay can take many forms. The most common format of bioassays based on chemiluminescence detection, consists in the use of a soluble molecule that is able to produce chemiluminescence when acted upon by an enzyme label coupled to a reporter molecule. This technology encompass the detection techniques based on horseradish peroxidase labels and luminol derivatives as substrates [Thorpe, G.H., Kricka, L.J. Methods Enzymol. 1986,133,331] or on alkaline phosphatase labels and dioxetanes as substrates [Olesen, C.E., Mosier, J., Voyta, J.C. , Bronstein, . Methods Enzymol. 2000, 305, 417]. The cyclic diacyl hydrazides, luminol, isoluminol and analogs such as naphthalene-1,2-dicarboxylic acid hydrazide, are co-substrates for horseradish peroxidase, and undergo a peroxidase-catalyzed oxidation in the presence of a suitable oxidant such as hydrogen peroxide or sodium perborate. This reaction was made commercially viable as an assay for peroxidase labels in enzyme immunoassay by the discovery that certain compounds increased and prolonged the signal in the presence of peroxidase, and at the same time decreased background light emission. Many types of molecule have been shown to act as enhancers including firefly luciferin, aromatic compounds, and various proprietary molecules contained in commercial reagents (e.g. SuperSignal, SuperSignal ULTRA and SuperSignal West Dura, see http://www.piercenet.com and WESTAR, WESTAR Nova and WESTAR SuperNova, see http://www.cyanagen.com ). The enhanced chemiluminescence assay principle has been commercialized as the VTTROS® ECi Immunodiagnostic System (see http://www.orthoclinical.com).

It is, however, often advantageous to directly label the reporter molecule with a compound that is chemiluminescent (probe). Labeling a reporter molecule with a compound that undergoes direct chemiluminescence upon induction offers the advantages of increased simplicity, increased rate of light generation, the potential for the attachment of multiple labels to the reporter, and the avoidance of having to attach large enzyme labels to the reporter molecule which can significantly affect its affinity for the target

This is the case of chemiluminescent immunoassays based on the use acridinium ester and sulfonamide labels [Pringle, M.J. Adv. Clin. Chem. 1993, 30, 89] or on luminol (3-aminophthalhydrazide) and isoluminol (5- aminophthalhydrazide) derivatives directly linked to proteins or antibodies (reporter molecules) [K. Van Dyke, R. Van Dyke. Luminescence Immunoassay and Molecular Applications, CRC Press, Boca Raton, 1990; D. Champiat, J.-P. Larpent, Bio-Chimi-Luminescence, Masson, Paris, 1993.]

In the assays based on the use of luminol derivatives, free or bound to a reporter protein, the chemiluminescence can also be obtained in the absence of the enzyme peroxidase.

In these cases, the excited aminophtalate ion (that emits luminescence) is formed from luminol through a reaction in solution catalyzed by ferriheme (Fe(III)porphyrins) derivatives that also act as enhancer, stabilizing the excited form [Jones, P., Frew, J.E., Scowen, N. J. Chem. Ed. 1987, 64 (1), 70-71; Jons, P, Wilson, I. Metal Ions in Biological Systems, Sigel, H. Ed. 1978, 7, 185]. Historically, luminol and isoluminol were the first chemiluminescent compounds to be used as labels to be conjugated to reporter molecules but they were quickly surpassed by the more sensitive acridinium esters labels [I. Weeks, I. Beheshti, F. McCapra, A.K. Campbell, J.S. Woodhead, Clin. Chem. 29 (1983) 1474].

The use of luminol and isoluminol as direct labels may result in a decrease in the efficiency and/or rapidity of light emission following covalent linkage to a reporter molecule. However, despite this, proteins, peptides and antibodies conjugated to isoluminol in the form of directly labeled probes have been successfully employed in a significant number of commercially available *in vitro* immunoassays such those marketed for use with DiaSorin's fully automated random access instrument, LIAISON (see http://www.diasorin.com).

A series of chemical methods can be used for the covalent attachment of the chemiluminescent molecule to the protein that are based on well established reactions for protein conjugation and labeling. The probes containing isocyanates, isothiocyanates, NHS esters or aldehydes can be linked to the amino groups of lysine residues, while thiol-reactive groups such as haloacetyl or maleimide groups and thiol-disulfide exchange reagents can be used for cysteine conjugation [Hermanson, G. T. Bioconjugate Techniques, Academic Press, San Diego,1996].

The great majority of amine-reactive cross-linking or modification reagents that are commercially available utilize *N-*hydroxysuccinimide (NHS) esters. An NHS ester may be formed by the reaction of a carboxylate with NHS in the presence of a carbodiimide. NHS or sulfo-NHS ester-containing reagents react with nucleophiles with release of the NHS or sulfo-NHS leaving group to form an acylated product. The NHS ester reacts with amino groups on the protein surface giving stable amide bonds in high yield, whereas the reaction of such esters with hydroxyl or sulfhydryl groups does not yield stable conjugates, forming ester or thioesters linkages, respectively. Both of these bonds hydrolyse in aqueous environments. Histidine side-chain nitrogens of the imidazoyl ring also can be acylated with an NHS ester reagent, but they too hydrolyze rapidly. Thus, in protein molecules, NHS ester reagents couple principally with the primary amines at the N-terminal and the secondary amines of lysine side chains.

NHS esters have an half-life of hours under physiological pH conditions, however both hydrolysis and amine reactivity increase with increasing pH. At 0°C at pH 7, the half-life is typically 4-5 h [Lomant, A.J., Fairbanks, G. J. Mol. Bio/. 1976,104, 243-261], at pH 8.6 and 4°C it falls to only 10 min [Cuatrecaseas, P., Parikh, I. Biochemistry 1972, 11, 2291-2299].

Despite their instability to hydrolysis, NHS esters of luminol and isoluminol derivatives, in particular the NHS active ester of *N*-(4-Aminobutyl)-*N*-ethylisoluminol (ABEI) with a succinate or glutarate linker are the current reagents of choice for the direct labeling of proteins, peptides and antibodies.

A more stable and pure *N*-(4-Aminobutyl)-*N*-ethylisoluminol (ABEI) labeling reagent would have a number of significant advantages from both an industrial process point of view in terms of ease of use and reproducibility of conjugation efficiency, and from a chemical conjugation standpoint in terms of increasing the number of ABEI molecules able to be linked to a given protein, peptide or antibodies, its incorporation efficiency.

### Advantages of the invention

The molecules of the invention allow a simple synthesis leading to higher chemical purity of the new ABEI anhydroglutarate (>99%) whereas ABEI-NHS shows purity ranging from 45%-60%.

Moreover the ABEI anhydroglutarate solution (in DMSO) is highly stable when stored at from +4°C to -30°C, whereas ABEI-NHS has to be stored in lyophilizxd form to be resolubilised in organic solvent just prior to use.

Further, the process of protein conjugation is simpler than ABEI-NHS.

Further, the ability to use DMSO in place of acetonitrile in the conjugation reaction causes less protein denaturation and potential aggregation, being DMSO a more protein friendly solvent.

As a result of both the higher purity and stability in aqueous solution an increased labeling of target proteins (higher incorporation capacity) is achieved.

As a result of both the higher purity and stability in both organic and aqueous solution, the obtained incorporation at a given molar ratio is more reproducible.

Finally, no or lower aggregation of conjugated proteins is achieved using ABEI anhydroglutarate vs ABEI-NHS at the same level of incorporation, as a result of:
(i) higher purity, less contaminants that may cause potential cross-linking reactions and
(ii) use of DMSO, a more protein friendly organic solvent, causing less denaturation which can lead to protein aggregation.

### Description of the invention

It is an object of the invention a compound of general formula (1) 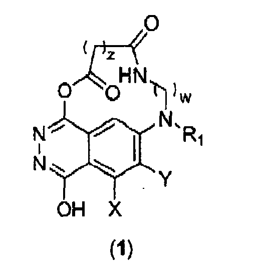 wherein:
X and Y, independently from one another, represent hydrogen, halide (Cl, Br, F, I), hydroxyl, primary or secondary amine, thiol, methoxy, alkyloxy group or alkyl group;
   - z is an integer number between 1 and 6;
   - w is an integer number between 2 and 6;
   - R₁ is C1-C5 alkyl.

Preferably X and Y are hydrogen, z = 2 or 3, w = 4 and R₁ = C2 alkyl (ethyl).

More preferably the present invention relates to the compound *N-*(4-Aminobutyl)-*N* ethylisoluminol anhydroglutarate (ABEI anhydroglutarate).

The compound is useful to covalently attach the chemiluminescent label *N-*(*4-*Aminobutyl)-*N-*ethylisoluminol (ABEI) to nucleophilic group containing molecules, as peptides and proteins, including antibodies, for the generation of labeled probes to be used in chemiluminescence-based immunoassays.

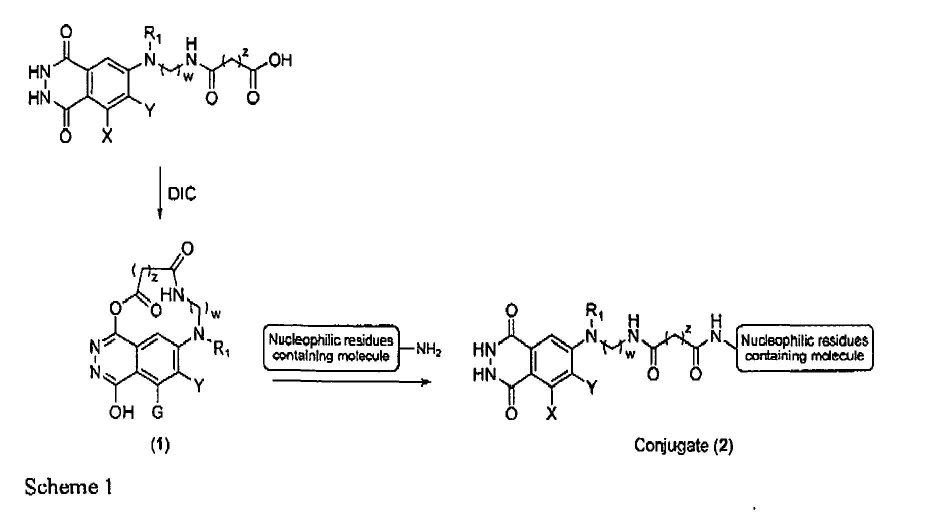

The compound of formula (1) is obtained by a one-step reaction from the ABEI-derivative by treatment with the condensing agent diisopropylcarbodiimide (DIC), according to Scheme 1.

Compound of formula (1) reacts with nucleophilic residues containing molecules, affording conjugates of general formula (2) in which the ABEI chemiluminescent label is linked to the nucleophilic residue containing molecule. Preferred nucleophilic residues containing molecules are lysine containing molecules, more preferably proteins, peptides, immunoglobulins and synthetic or recombinant derivatives thereof. While having a reactivity with such molecules similar to that of the equivalent *N-* hydroxysuccinimide (NHS) ester, compound of formula (1) is more stable to hydrolysis and can be handled and stored more easily. The chemiluminescence properties of the protein-ABEI' conjugates were analyzed and the two methods for ABEI labeling based on the use of the NHS active ester or molecule (1) were compared. The superior stability to hydrolysis makes compound of formula (1) a good candidate to replace the NHS esters in the preparation of probes (proteins, antibodies) labeled with ABEI.

The compound of formula (1) reacts with nucleophilic residue containing molecules, as amino groups of proteins/peptides/lysine containing derivatives, affording products of general formula conjugate (2) in which the chemiluminescent label N-(4-Aminobutyl)-N-ethylisoluminol (ABEI) is covalently attached to the molecule through a linker by means of the following reaction:

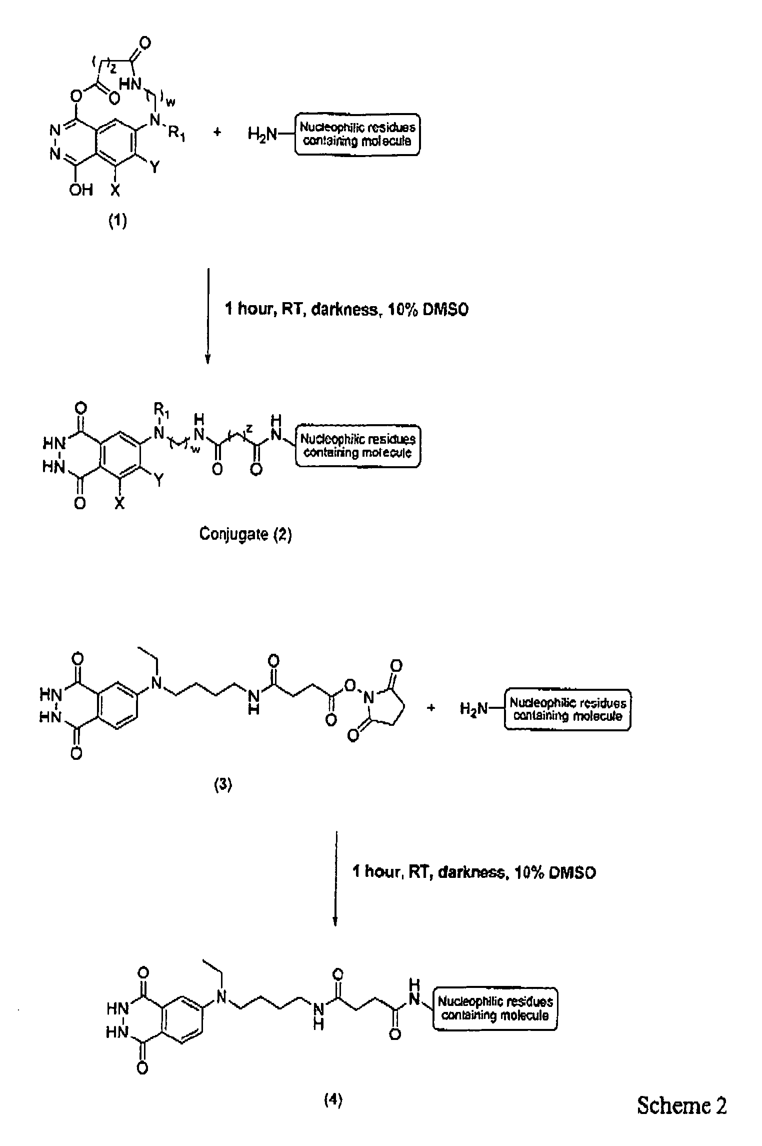

Compound (1a), namely *N*-(4-Aminobutyl)-*N-*ethylisoluminol anhydroglutarate (ABEI anhydroglutarate), is prepared by reacting ABEI glutarate with the condensing agent diisopropylcarbodiimide (DIC) in the solvent dimethylformamide (DMF), according to the following reaction:

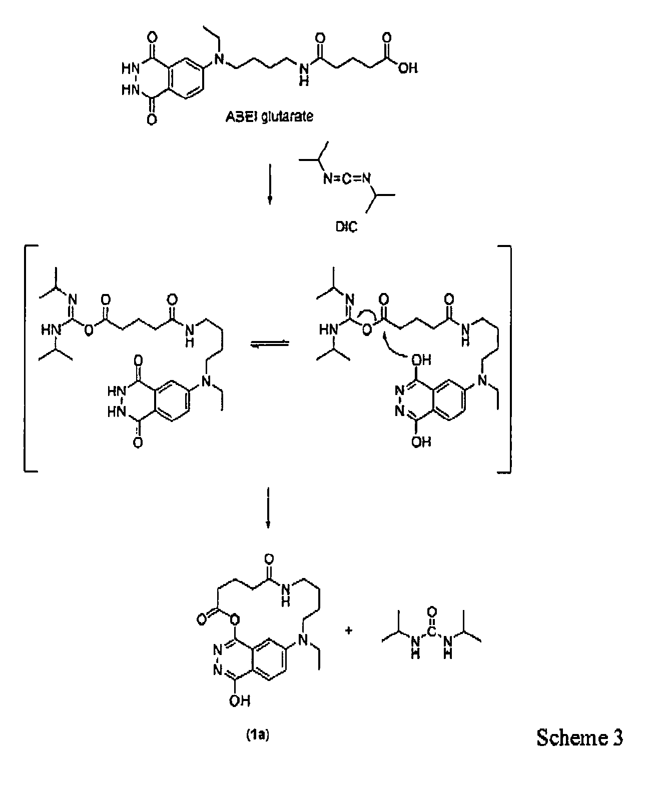

Once the key intermediate *O*-acylisourea adduct is formed, nucleophilic attack of the oxygen of the aromatic form of ABEI to the activated carboxylic group, with subsequent elimination of urea, leads to the formation of compoud of formula (1a) by intramolecular cyclisation.

The same synthetic pathway is applied to the synthesis of the ABEI-succinate compound (1,wherein X and Y are hydrogen, z = 2, w = 4 and R₁ = C2 alkyl), although with lower yields, probably due to a less favorable cyclization step.

The formation of cyclic products has never been reported for ABEI derivatives, however, a very similar nucleophilic attack of enamide oxygen to activated acyl groups has been described in intermolecular reactions such as the synthesis of some acyl luminol derivatives [Y. Omote, T. Miyake, S. Ohmori,N. Sugiyama Bull. Chem. Soc. Jpn. 1996, 39,932-935].

Compound of formula (1a) has a reactivity similar to NHS or sulfo-NHS esters with compounds containing a primary amino group: the amine opens the cyclic structure and is finally covalently linked to ABEI through a glutaramide linker. This reactivity was verified with simpler amine models such as benzylamine. Compound of formula (1a) could therefore be used to label any reporter molecules that bear an amino group for the reaction. In contrast with NHS active esters (3) (Scheme 2) and other active esters that are rapidly hydrolyzed during chromatographic processes on silica gel, compound of formula (1a) is stable enough to be purified by direct phase flash chromatography and can be obtained with a purity of 98% or more.

Four different preparations of compound of formula (1a) *N-*(4-Aminobutyl)-*N-*ethylisoluminol (Scheme 4) were made (50-250 mg scale) followed by flash-chromatography purification of the product, obtaining reproducible values of reaction yield and product purity.

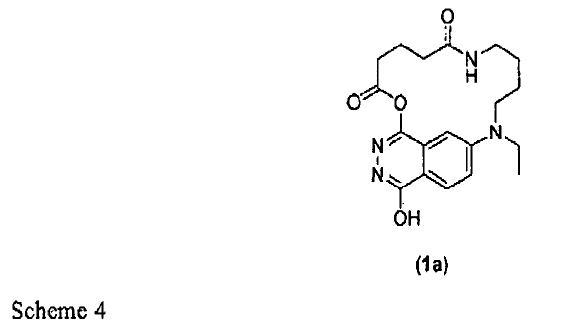

The chemical structure of compound of formula (1a) was determined by means of NMR (mono and bidimensional experiments on hydrogen and carbon), mass, and IR analysis (described in detail in the experimental section).

Compound (1a) reacts with protein in the absence of condensing agents, its reactivity is similar to that of the corresponding ABEI succinate active ester, in particular the N-hydroxysuccinimide (NHS) (3) or sulfo-NHS ester affording a conjugation product of formula (4) with an almost identical glutaramide linker, only differing by a single additional carbon atom in the linker arm.

It is another object of the invention a chemiluminescent conjugate consisting of a first molecule containing a nucleophilic group and a second molecule being the compound of formula (1) said second molecule being covalently attached to the nucleophilic group of said first molecule. Preferably said first molecule is selected among a primary amine, a secondary amine, a thiol or an alcohol. More preferably the first molecule is a molecule containing at least one lysine residue and the second molecule is covalently attached to the amino terminal of said lysine residue.

In a particular aspect of the invention the lysine residue containing molecule is a protein, a recombinant protein, a peptide or a synthetic peptide derivative. In a further aspect the lysine residue containing molecule is an immunoglobulin or a synthetic or recombinant derivative thereof, preferably able to specifically bind to a pathogen derived antigen.

In a preferred aspect the immunoglobulin or a synthetic or recombinant derivative thereof is able to specifically bind to a HBV antigen, preferably HBsAg.

Alternatively the immunoglobulin or a synthetic or recombinant derivative thereof is able to specifically bind to a HCV antigen.

Alternatively the immunoglobulin or a synthetic or recombinant derivative thereof is able to specifically bind to a HIV antigen, preferably HIV gp41.

It is another object of the invention the use of the compound as above disclosed to label a molecule containing a nucleophilic group and obtaining a chemiluminescent conjugate.

It is another object of the invention the use of the compound according as above disclosed to label a molecule containing at least one lysine residue and obtaining a chemiluminescent conjugate.

It is another object of the invention an immunoassay for the detection of an antigen that makes use of the chemiluminescent conjugate of the invention as labeled probe.

It is another object of the invention an immunodiagnostic kit comprising the chemiluminescent conjugate of the invention as labeled probe.

### Brief description of the figures

**Figure 1****: HPLC analysis of cyclization reaction: RP-HPLC, C18 column (Symmetry,** 4.5x250 mm, Waters), instrument Jasco PU-2080, grad. 0%-24% of A in 10 min, 24% for 10 min, 24% -100% of A in 5 min, 5 min 100 % of A. Eluent A: 98 % CH₃CN + 2% H₂O + 0.1% TFA; Eluent **B**: 98% H₂O + 2% CH₃CN + 0.1 % TFA, flow 1 mL/min, detection @280 nm, reaction sample (5 microL dil. 1:20 in DMSO) after 0h, 48h and 120h.
Figure 2: ¹H-NMR. Spectrum of compound **1a** (10 mM,d₆-DMSO) was acquired at 300 K. Spectrum description: ¹H-NMR (400 MHz, dDMSO) δ 12.00 (s, 1H, *H11),* 8.10 (t, J= 6.1, 1H, *H20),* 7.98 (d, J= 9.1, 1H, *H3),* 7.22 (dd, J= 9.2, 2.5, 1H, *H4*),6.40 (d, *J*= 2.4, 1H, *H6),* 3.51 (q, *J* = 6.9, 2H, *H14),* 3.32 (m, 2H, *H15*), 3.08 (bm, 2H, *H19),* 2.77 (t, *J* = 6.0, 2H, *H25),* 2.29 (bt, 2H, *H22),* 1.82 (bm, 2H, *H24),* 1.55 (bm, *J*= 5.6, 2H, *H17),* 1.41 (bm, 2H, *H18),* 1.13 (t, J= 7.0, 3H, *H16).*
**Figure 3**: ¹³**C-APT NMR**. Spectrum of compound **1a** (20 mM, d₆-DMSO) was acquired at 300 K. Spectrum description: ¹³C-NMR (100 MHz, dDMSO) δ 170.98, 170.78, 159.44, 150.87,144.08,128.22,127.35,116-71,116.27,100.15,50.53,44.87,37.89,32.01,29.53, 26.69, 23.33, 23.21,18.75, 12.54
**Figure 4****: 2D-TOCSY.** Spectrum of compound **1a** (10 mM, d₆-DMSO) was acquired at 308 K.
**Figure 5****: GCOSY.** Spectrum of compound **1a** (10 mM, d₆-DMSO) was acquired at 300 K.
**Figure 6****: 2D-NOESY.** Spectrum of compound **1a** (5 mM, d₆-DMSO) was acquired at 308 K and 0.6 s of mixing time. The evidence of different proton interactions were showed as different color sets in the upper figure.
**Figure 7****:** **¹****H-¹³C gHSQC.** Spectrum of compound **1a** (20 mM, d₆-DMSO) was acquired at 300 K.
**Figure 8****: N*H*20** chemical-shift dependence on temperature. ¹H-NMR analysis of a solution of compound **1a** (10mM, d6-DMSO) was performed at different temperature (293, 303, 313,323,333 and 343 K), and the correlation chemical-shift temperature Δδ(NH)/ΔT was calculated on N*H*20 peak.
**Figure 9****:** long-term stability of the ABEI anhydroglutarate in solution (DMSO) stored at - 30°C.
**Figure 10****:** long-term stability of the ABEI anhydroglutarate in solution (DMSO) stored at 4°C.
**Figure 11****:** Immunoassay model 1 analysis of reactivity (RLU) of serially diluted samples using selected biotinylated Fab fragment (solid phase) and sheep polyclonal antibodies to detect HBsAg: Comparison between ABEI-NHS tracers versus ABEI anhydroglutarate tracers derived from different labeling molar ratios (10x, 20, 40x) is reported; standard samples from A to C.
**Figure 12****:** Immunoassay model 1 analysis of reactivity (RLU) of serial diluted samples using selected biotinylated Fab fragment (solid phase) and sheep polyclonal antibodies to detect HBsAg: Comparison between ABEI-NHS tracers versus ABEI anhydroglutarate tracers derived from different labeling molar ratios (10x, 20x, 40x) is reported; standard samples from D to H.
**Figure 13****: Immunoassay model 1 analysis of specificity (RLU) of a small sample** population of blood donors (20 samples); the RLU values are related to mean of negative samples, highest negative sample, minimum negative sample and standard deviation of the negative sample population is reported.
**Figure 14****: I**mmunoassay model 2 analysis of reactivity (RLU) of serially diluted samples using selected mouse monoclonal antibody (solid phase) and a second monoclonal antibody to different epitope for detecting HBsAg: comparison between ABEI-NHS tracers versus ABEI anhydroglutarate tracers derived from different labeling molar ratios (10x, 20x, 40x) is reported; standard samples from A to C.
**Figure 15****:** Immunoassay model 2 analysis of reactivity (RLU) of serial diluted samples using selected mouse monoclonal antibody (solid phase) and a second monoclonal antibody to different epitope for detecting HBsAg: comparison between ABEI-NHS tracers versus ABEI anhydroglutarate tracers derived from different labeling molar ratios (10x 20x, 40x) is reported; standard samples from D to H.
**Figure 16**: Immunoassay model 2 analysis of specificity (RLU) of a small sample population of blood donors (20 samples); the RLU values is related to mean of negative samples, highest negative sample, minimum negative sample and standard deviation of the negative sample population.
**Figure 17****:** Immunoassay model 3 analysis of reactivity (RLU) of IgG to HIV gp41 of serial diluted sample 502. Comparison between ABEI-NHS (25x molar ratio conjugation) tracer versus ABEI anhydroglutarate (25x molar ratio conjugation) tracer is reported.
**Figure 18****:** Immunoassay model 3 analysis of reactivity (RLU) of IgG to HIV gp41 of serial diluted sample 504. Comparison between ABEI-NHS (25x molar ratio conjugation) tracer versus ABEI anbydroglutarate (25x molar ratio conjugation) tracer is reported.
**Figure 19****:** Immunoassay model 3 analysis of specificity (RLU) of a very little sample population of blood donors (3 samples). Comparison between ABEI-NHS (25x molar ratio conjugation) tracer versus ABEI anhydroglutarate (25x molar ratio conjugation) tracer is reported.
**Figure 20**: Immunoassay model 3 analysis of reactivity (RLU) of IgG to HIV gp41 of serial diluted samples 502 after different thermal stress treatment of the tracer. Comparison between ABEI-NHS (25x molar ratio conjugation) tracer versus ABEI anhydroglutarate (25x molar ratio conjugation) tracer is reported.
**Figure 21****:** Immunoassay model 3 analysis of reactivity (RLU) of IgG to HIV gp41 of serial diluted sample 504 after different thermal stress treatment of the tracer. Comparison between ABEI-NHS (25x molar ratio conjugation) tracer versus ABEI anhydroglutarate (25x molar ratio conjugation) tracer is reported.
**Figure 22****:** Immunoassay model 3 analysis of specificity (RLU) of a very small sample population of blood donors (3 samples) after different thermal stress treatment of the tracer ABEI anhydroglutarate conjugate. Comparison between ABEI-NHS (25x molar ratio conjugation) tracer versus ABEI anhydroglutarate (25x molar ratio conjugation) tracer is reported.

### EXPERIMENTAL SECTION

### Chemistry

### General procedures

All solvents were dried over molecular sieves (3 - 4 Å, Fluka) for at least 24 h before use or purchased by Sigma-Aldrich with a content of water ≤0.005 %. When anhydrous conditions were required, the reactions were performed under argon atmosphere. Thin-layer chromatography (TLC) was performed on Silica Gel 60 F254 plates (Merck) with UV detection, or using a developing solution of conc. H₂SO₄/EtOH/H₂O (5:45:45), followed by heating at 180°C. Flash column chromatography was performed on silica gel 230-400 mesh (Merck). Mixtures of petroleum ether (boiling range 40-60°C) and ethyl acetate were used as eluent. ¹H and ¹³C NMR spectra were recorded on a Varian 400 MHz MERCURY instrument at 300 K. Chemical shifts are reported in ppm downfield from TMS as internal standard. Mass spectra were recorded on ESI-MS triple quadrupole (model API2000 QTrap™, Applied Biosystems). High resolution mass spectra were recorded on QSTAR Elite® LC/MS/MS system (Applied Biosystems) that is a hybrid quadrupole/TOF instrument, equipped with the Analyst® QS 2.0 software.

### Synthesis of N-(4-Aminobutyl)-N-ethylisoluminol anhydroglutarate (ABEI anhydroglutarate) (1a).

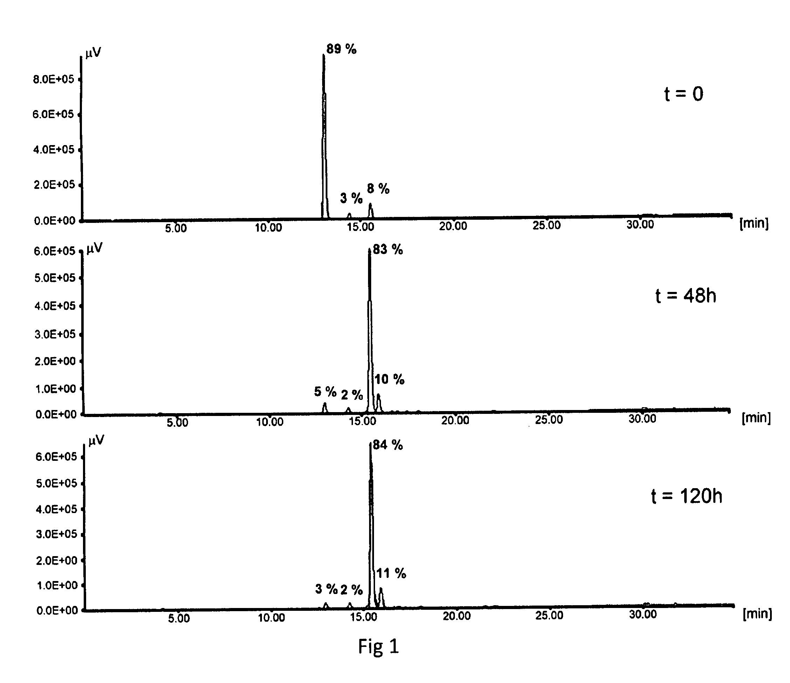

**Procedure A**: to a solution of glutaric anhydride (217 mg, 1.90 mmol, 1.75 eq) in anhydrous pyridine (15 mL), commercial ABEI (Sigma A0156) (300 mg, 1.08 mmol, 1 eq) was added under argon atmosphere. The solution was warmed at 50°C for 10 min, until ABEI was completely dissolved, then kept at this temperature for another 10 min. After this time, TLC analysis (eluent: CHC/MeOH/AcOH = 7:3:0.05, UV detection 254 nm) revealed that reaction was complete (R_{f} ABEI = 0, R_{f} ABEI-glutarate = 0.56). Solvent was evaporated under vacuum and the crude intermediate product was purified by flash chromatography (eluent: CHCl₃/MeOH/AcOH = 9:1:0.05) obtaining ABEI-glutarate as a light yellow solid in a quantitative yield. The residue was then dissolved in anhydrous DMF (25 mL) and DIC (251 µL, 1.62 mmol, 1.5 eq) was added at 0°C. The reaction mixture was stirred at 25°C and was followed by HPLC analysis (Fig. 1). After 48-72 hours the reagent was almost totally converted into product, compound **1a.** Subsequently the solvent was evaporated under vacuum and the crude product, compound **1a,** was purified by flash chromatography (linear gradient of eluent CH₃Cl to CH₃Cl:MeOH. 10:0.5). Compound **1a** was obtained as a white solid (160 mg, yield 40%) having NMR characteristics: ¹H-NMR (400 MHz, dDMSO) δ 12.00 (s, 1H, *H11),* 8.10 (t, J= 6.1, 1H, *H20),* 7.98 (d, *J=* 9.1,1H, *H3),* 7.22 (dd,*J=* 9.2, 2.5,1H, *H4),* 6.40 (d, *J=* 2.4, 1H, *H6*)*,* 3.51 (q, *J*= 6.9, 2H, *H14),* 3.32 (m, *2H,H15),* 3.08 (bm, 2H, *H19),* 2.77 (t, *J =* 6.0, 2H, *H25),* 2.29 (bt, 2H, *H22),* 1.82 (bm, 2H, *H24),* 1.55 (bm, *J -* 5.6, 2H,*H* 17), 1.41 (bm, 2H, *H18),* 1.13 (t,*J*=7.0.3H,*H16).* ¹³C-NMR (100 MHz, dDMSO) δ 170.98,170.78.159.44, 150.87, 144.08, 128.22, 127.35,116.71,116.27,100.15, 50.53,44.87, 37.89, 32.0!, 29.53, 26.69, 23.33, 23.21, 18.75, 12.54. FT-IR(cm⁻¹) 1774.828, 1590.170, 1512.354, 1407.959, 1921.315, 1285.770, 1118.480, 1091.894, 909.528, 877.127, 797.415. HRMS(FT-ICR):calcd for C₁₉H₂₄N₄O₄: 372.41.83 found: 373.1795 (M+H)⁺, 395.34215 (M+Na)⁺, 411.2753 (M+K)⁺, 371.3964 (M-H). Elemental analysis: calcd for C₁₉H₂₄N₄O₄: C 61.28 %; H 6.5 %; N 15.04 %, found: C 59.91%; H 6.44 %; N 13.97 %.

**Procedure B**: a solution of ABEI (250 mg, 0.9 mmol, 1 eq) and glutaric anhydride (103 mg, 0.9 mmol, 1 eq) in 10 mL of DMSO was stirred at 37 ± 5°C until it became homogeneous (about 5-10 minutes), then stirred at room temperature protected from light for an additional 30 - 60 minutes. EDC (260 mg, 1.5 eq) was added and the resulting mixture was stirred overnight at room temperature, protected from light. After this time LCMS analysis (Analytical C₁₈ Column 2.1 x 50 mm, PDA @ 322 nm, m/z = 373 Es⁺) revealed product formation≥ 65%. The ABEI-glutarate reaction mixture was added rapidly by dropper pipette to a 500 mL Erlenmeyer flask containing 300 mL of vigorously stirring ethyl acetate and mixed for 20. The mixture was then transferred to a 500 mL separatory funnel and washed with 75 mL of saturated sodium chloride solution. The organic layer was further washed with deionized water (25 mL x 2) and dried with Na₂SO₄. The solvent was evaporated using a rotary evaporator with a water bath temperature of 55°C to 65°C. Compound **1a** was purified by crystallization from MeOH and the collected solids were dried under a stream of nitrogen in a heating block set at 50°C and lyophilized overnight. Compound **1a**, obtained in 45 % yield, was identical by HPLC, PDA and mass spectral characterization to the compound obtained by procedure A.

### Structure determination.

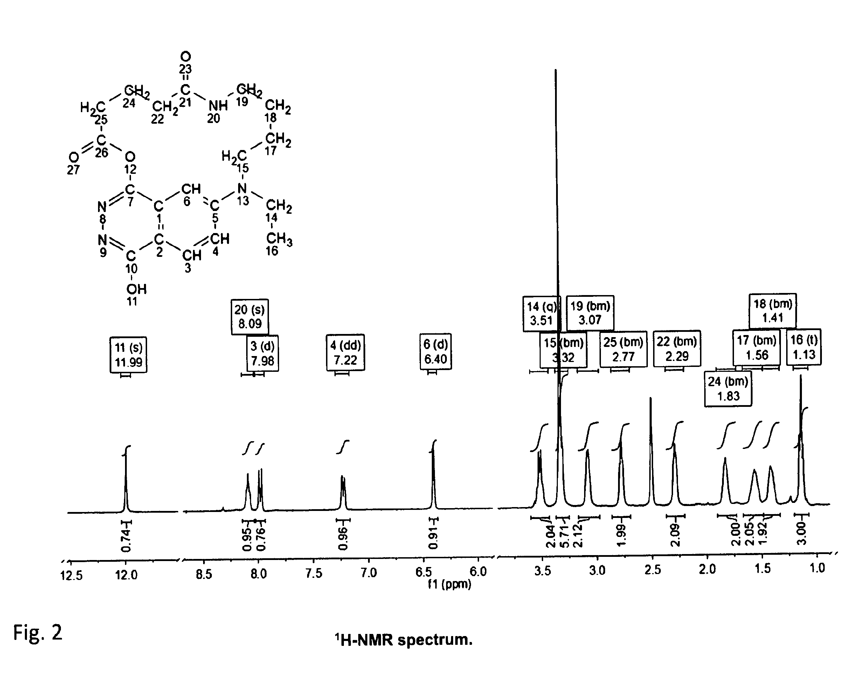

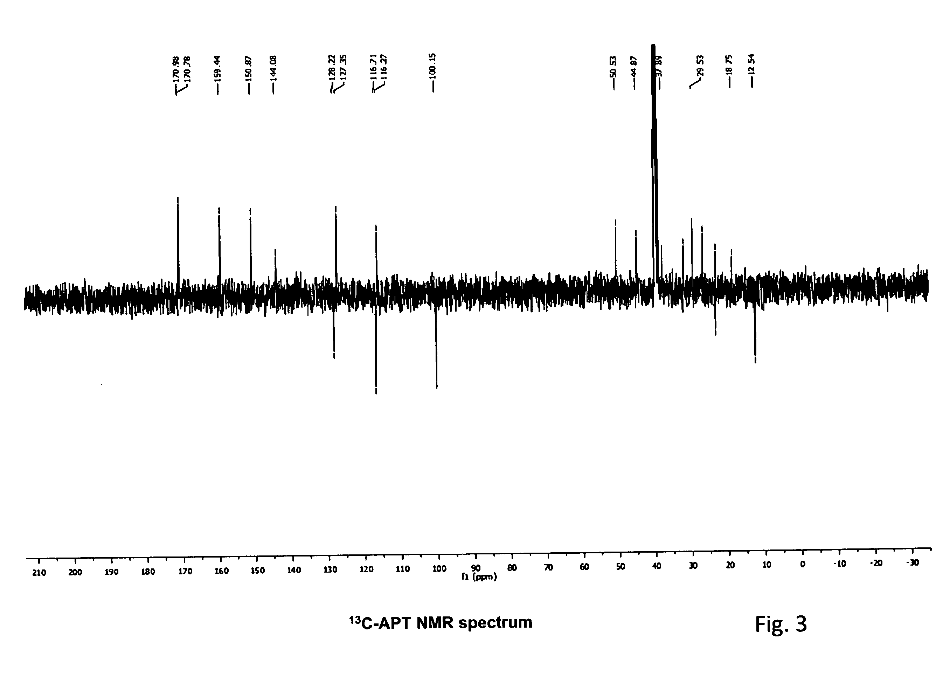

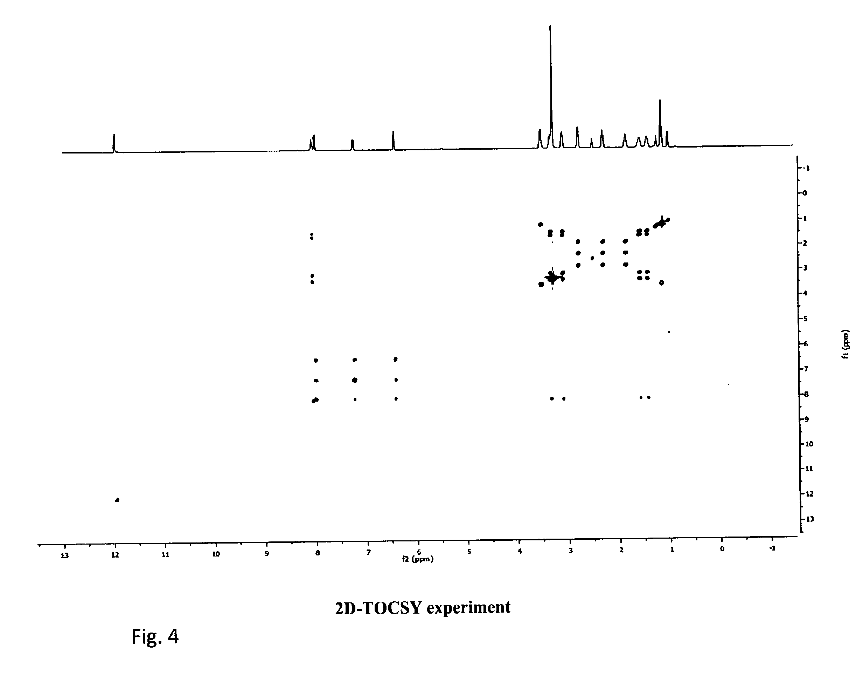

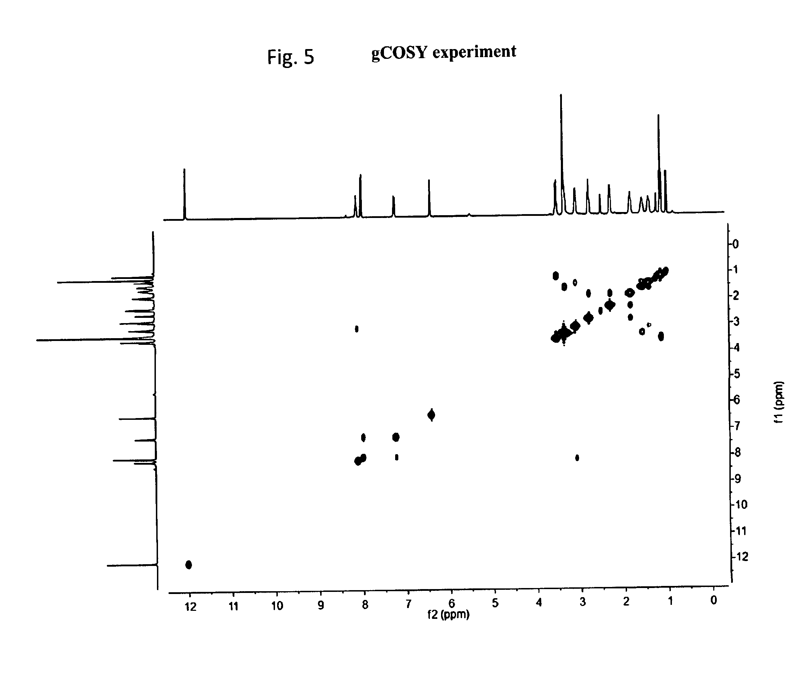

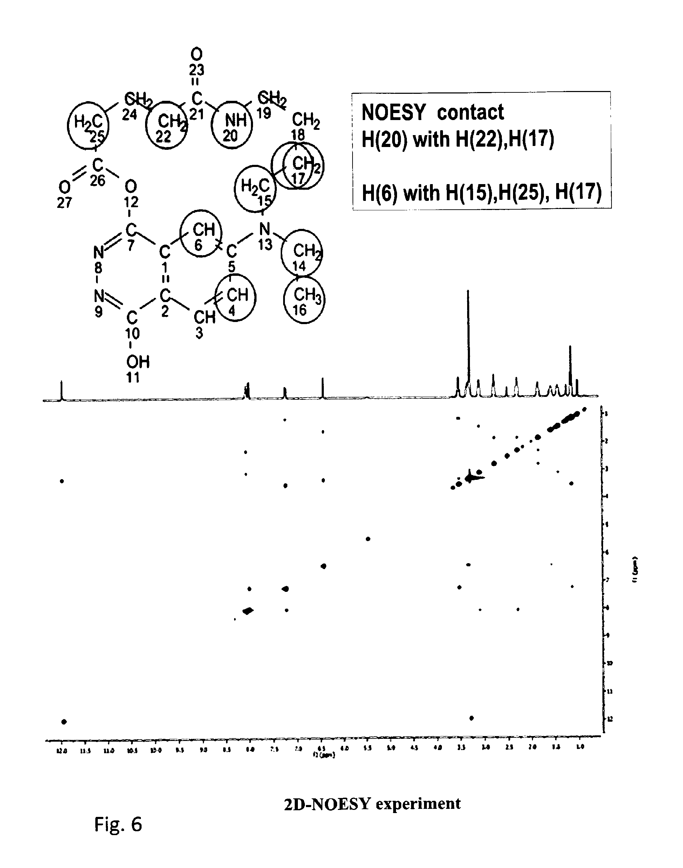

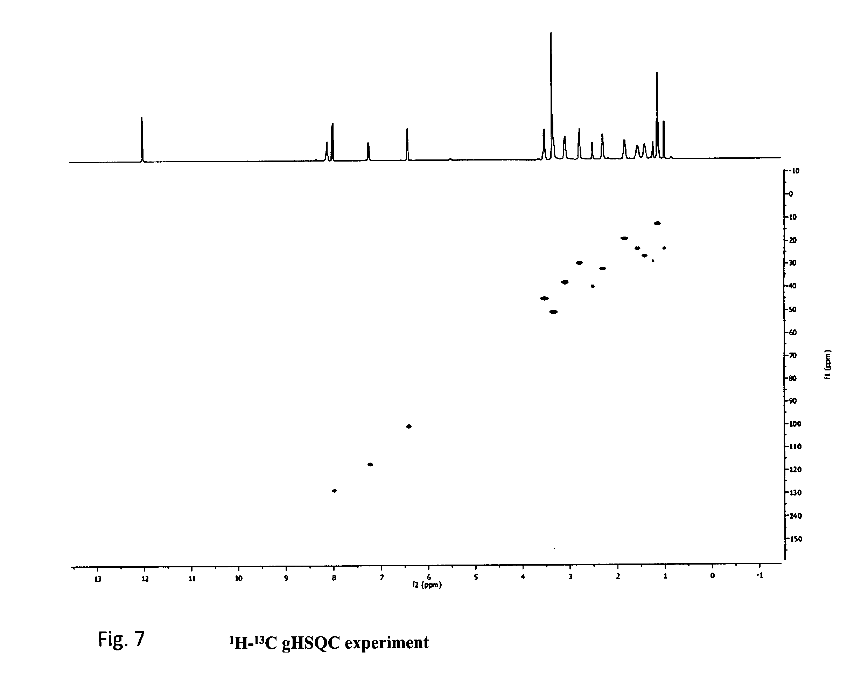

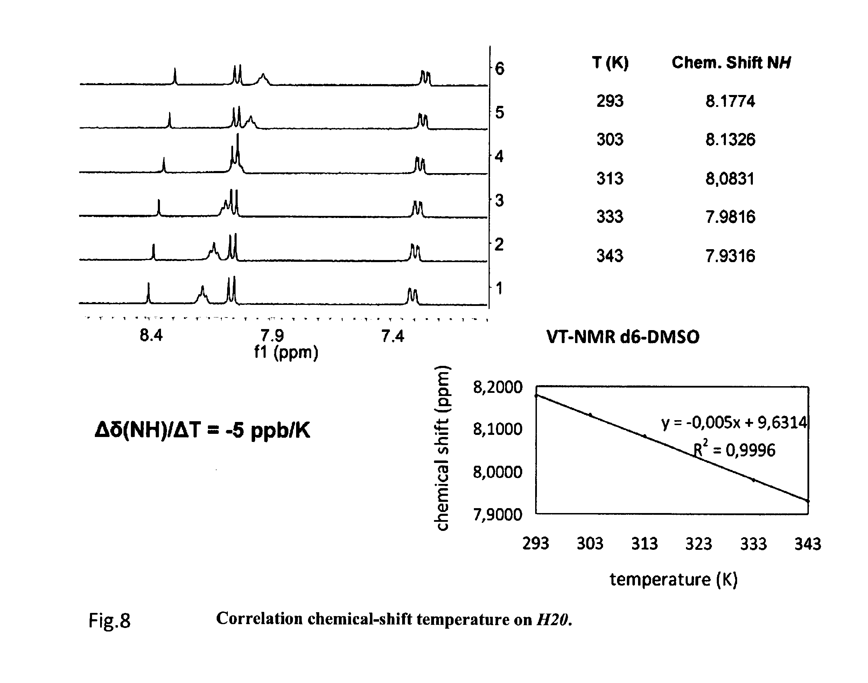

The structure of compound of formula **1a** was determined on the basis of 1D and 2D (¹H, ¹³C APT, gCOSY, 2D-TQCSY, ¹H-¹³C gHSQC, 2D-NOESY) NMR experiments, (figs. 2, 3, 4, 5, 6, 7 and 8). ¹H-NMR spectrum shows a sharp signal at 12 ppm, involved in chemical exchange with deuterium in presence of D₂O, assigned to enolic proton *H11*. 2D-NOESY experiments, performed at different mixing times at 35°C, show three sets of interactions between *H20, H22* and *H17,* between *H4, H14* and *H16* and between *H6, H15, H25* and *H17* that supported the cyclic structure (Fig. 6). In addition, the conformational rigidity of compound **1a** was confirmed by *H14, H17, R18, H19, H22, H24* and *H25* line broadening. Interesting, ¹H-NMR experiments at variable temperature (20-70°C) show a temperature-dependent shift of the signal of the *NH* proton (*H20*). The calculated Δδ(NH)/ΔT= -5ppb/K indicates that this proton is involved in an intramolecular hydrogen bond shorter than 2Å (Cierpicki, T.; Otlewski, J., Amide proton temperature coefficients as hydrogen bond indicators in proteins. Journal of Biomolecular NMR 2001, 21 (3), 249-261) (Figure S8). The intramolecular nature of this bond is also confirmed by ¹H-NMR experiment at variable concentration (data not show), as N*H20* chemical shift is not concentration-dependent. Mass spectrometry analysis was performed both for positive and negative ions. Mass analysis of sample in acid medium (0.04 mM in 90% water, 10% DMSO and 0.1% formic acid) shows a peak at 373.2 Da corresponding to protonated compound of formula 1, Moreover, mass analysis in basic medium (0.04 mM in 90% water, 10% DMSO, 0.1% Et₃N) shows a peak at 371.4 correspondig to the deprotonated compound. It represents a further evidence of the presence of enolic species. Finally the strong infrared absorption observed at 1770 cm⁻¹ is characteristic of the carbonyl group of the enol ester type.

The stability of compound (**1a**) dissolved in organic solvents (DMSO, acetonitrile) was evaluated at two different storage temperatures, -30°C and +4°C, by monitoring the formation of the hydrolysis product (ABEI-glutaric acid) using a chromatographic HPLC system.

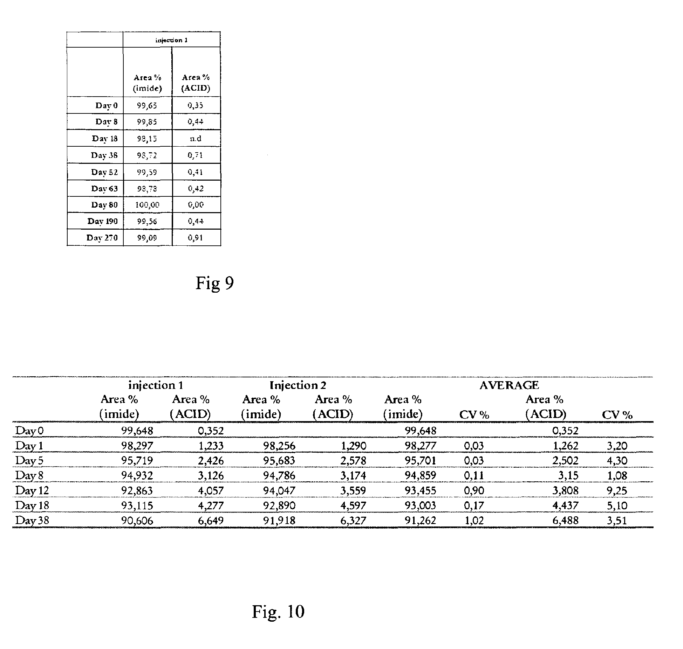

Within the limits of reproducibility of the experimental determination, less than 2% of compound (**1a**) was hydrolysed to glutaric acid after 270 days at -30°C (Fig. 9), while less than 7% of compound (**1a**) was hydrolysed to acid at +4°C after 38 days (Fig. 10). This compares to the reported high instability of equivalent NHS esters which have half-lives of hours under physiological pH conditions. At 0°C and at pH 7, the half-life is typically 4-5 h [Lomant, A.J., Fairbanks, G. J. Mol. Biol. 1976, 104, 243-261], at pH 8.6 and 4°C this falls to only 10 min [Cuatrecaseas, P., Parikh, I. Biochemistry 1972, 11, 2291-2299].

### Preparation of ABEI anhydroglutarate solution and aliquots

The anhydrous powder of ABEI anhydroglutarate was removed from the freezer, -30°C, and left to equilibrate at room temperature for 60 minutes. Anhydrous DMSO was added to the ABEI anhydroglutarate powder to obtain a 2 mg/ml solution. The sample was vortexed several times for few seconds each time while being kept at +4°C. ABEI anhydroglutarate reagent solution was quickly divided into aliquots of 100 µl each which were then immediately stored at -30°C. These aliquots were the used once only and discarded.

### General procedure for ABEI anhydroglutarate labeling reactions

1. Proteins to be labeled must be present in a buffer free of amino groups (e.g. not Tris) at a concentration between 1 and 2 mg/ml. If the protein buffer contains free amino groups, they must be dialysed extensively against "dialysis-dilution-elution pH 7,40 buffer, **buffer B** " (see below Appendix), using several buffer changes. If the protein solution is less than 1 mg/ml, it may be necessary to concentrate it with any appropriate device, e.g. ultra filtration membranes. Alternatively if the protein concentration is higher than 3-4 mg/ml is preferable to dilute it with **buffer B** (see below "dialysis-dilution-elution pH 7,40 buffer").
2. In a 5ml brown glass vial (the reaction tube) add in sequence the calculated volumes of the dilution buffer, the 10x reaction buffer, the DMSO anhydrous solution, the protein solution and finally the solution of ABEI anhydroglutarate 2 mg/mL by following the example depicted in the box 1.
   - At the beginning the following reaction parameters need to be fixed:
      ■ Amount of protein to be labeled, where **Cₚ** is the protein concentration before labeling (mg/mL) and **P** is the protein volume to be used (mL)
      ■ Protein concentration in the labeling reaction. **C_{R}** (mg/mL)
      ■ Molar excess of ABEI-anhydrogluatarate versus the mole of protein to be reacted. **T**
   - Add to the chosen protein volume the correct volume of diluent buffer (**buffer B**) to bring the protein concentration down to the prefixed label protein concentration. Final reaction volume **X** (mL)= (**P * Cₚ**)/**C_{R}**
   - Add to the diluted protein a volume of **buffer A** (see below Appendix, 0,1 1M Phosphate pH 8,90 "conjugation" buffer) equal to 1/10 of volume X. Final reaction volume= **1.1 *X**
   - Calculate required volume of ABEI anhydroglutarate solution depending on the molar ratio excess that is required; Added volume **Y**.
   - Add DMSO to have a 9% of organic content in the reaction mixture. Volume of added DMSO (**Z**)= (0-09***1.1*X)-Y**
      Final reaction volume: **1.1*X + Y + Z**
3. Incubate the reaction mixture at room temperature (17-24°C) for 60 minutes in the dark.
4. The resulting reaction mixture is then filtered 0.45 µm (Millex-HV, Millipore) and the conjugated protein purified from unreacted reagents by gel filtration chromatography (Superdex 200 GE) using buffer B (dialysis-dilution-elution pH 7,40 buffer) and collecting those fractions corresponding to the anticipated molecular weight of the protein while simultaneously recording the absorbance spectrum at 280 and 329 nm.
5. Protein-ABEI conjugates should be stored in tracer storage buffer (see Appendix below).

### Box 1 : Calculation example:

*Starting with extensively dialyzed protein (MW 45 KD) 1 mix 3 mg*/*ml = 3 mg*
*First dilute to around 1,0 mg*/*ml :*
*Volume :* 3 *ml (X volume)*
*Add 3*/*10 = 0,3 ml of buffer A( 0,1M Phosphate pH 8,90)*
*New volume : 3,3 ml (1.1*X volume)*
*Required organic phase volume : = 0,297 ml (0.09*1.1*X volume)*
*Required ABEI anhydroglutarate, if a molar excess of 15 : 1 is used (ABEI anhydroglutarate MW : 372 ; concentration is assumed = 2 mg*/*ml)*
*(3 mg*/*45000) * 15 * 372 = 0,372 mg*
*0.372 mg*/*2 mg*/*ml = 0,186 ml (Y volume)*
*DMSO to be added to have around 9% organic phase :*
*0,297 - 0.186 = 0,111 ml (Z volume)*

### ABEI Incorporation Evaluation

The degree of incorporation (conjugation) of ABEI to a protein, peptide or antibody can be calculated in relative terms by measuring the ratio of the wavelengths at 280nm (main contribution from protein) and 329nm (main contribution from ABEI) of the peak of the protein exiting from the gel filtration column. This can be performed either as a ratio of the absorbances 280nm/329nm at the maximum peak height of the curve or as a ratio of the integrated areas of absorbances 280nm/329nm under the curves for the conjugate peak. This ratio, Fh or Fa depeding on whether height or area is used respectively, is inversely proportional to the amount of ABEI incorporated (higher values = less ABEI, lower values = more ABEI)
- **Buffer A; 0,1M sodium phosphate pH 8,90 buffer ("conjugation buffer")**
   (please note that this buffer must be stored at temperature below +30 °C ,otherwise precipitation happens) *Na₂HPO₄*2H₂O 0,1 M : Adjust pH to 8,90 with NaOH*
- **Buffer B; PBS pH 7,40 buffer ("dialysis-dilution-elution buffer") :**
   *1,8 mM KH₂PO₄; 8,2 mM Na₂HPO₄*2H₂O: 145 mM NaCl; adjust pH to 7,40 with NaOH or H₃PO₄.*
- **PBS - Sodium Azide - BSA pH 7,20 buffer ("tracer storage buffer")**
   *0,372g*/*L KH2PO4; 1,292g*/*L Na₂HPO₄*2H₂O; 0,2 g*/*L KCl; 8,0 g*/*L NaCl; 0.9 g*/*L NaN₃; 1,0 g*/*L BSA; adjust pH to 7,20 with NaOH*

The ability of the new ABEI anhydroglutarate to efficiently conjugate to a variety of proteins was tested with a number of model systems. In each case the protein was also conjugated under standard conditions with ABEI-NHS, Analytical immunometric comparison between the ABEI-NHS tracers and ABEJ anhydroglutarate tracers was performed using the Liaison immunoassay instrument platform (DiaSorin). For each immunoassay model system the relative light unit (RLU) value coming from each sample is the mean of 3 replicates; only results having RLUs with a CV lower than 15% were considered valid.

### PRINCIPLE OF THE ASSAY n.1

The method for qualitative HBsAg determination is a direct sandwich chemiluminescence immunoassay (CLIA).

Antibodies to HBsAg (biotinylated Fab fragment mouse monoclonal antibodies) are used for coating paramagnetic particles (solid phase) and sheep polyclonal antibodies to HBsAg were conjugated to ABEI anhydroglutarate (tracer) or ABEI-NHS (tracer) using the molar ratios 10x 20x, 40x.

During the first incubation, HBSAg present in samples binds to the solid phase. During the second incubation, the antibody conjugate reacts with HBsAg already bound to the solid phase. After the second incubation, the unbound material is removed with a wash cycle. Subsequently, starter reagents are added and a flash chemiluminescence reaction is induced. The light signal, and hence the amount of ABEI anhydroglutarate-antibody conjugate, is measured by a photomultiplier as relative light units (RLU) and is indicative of HBsAg the concentration present in the samples.

Apart from the tracers (sheep polyclonal antibodies conjugated with ABEI anhydroglutarate and ABEI-NHS), the assay reagents working concentration and assay methods were the same as for the Liaison HBsAg kit (DiaSorin cat #310100).

We used as positive samples a standard curve (8 samples from A till H) at known concentrations of HBsAg obtained by serial dilution in negative serum of high positive HBsAg sample. The dilutions were made to get the measured value within the linearity range of the Liaison HBsAg expressed as Index value.

The index Value of the obtained standard curve is the following:
Standard A: > 240 RLU 99764
Standard B: 108 RLU 35963
Standard C: 71 RLU 23562
Standard D: 26 RLU 10060
Standard E: 19 RLU 7677
Standard F: 8.0 RLU 3168
Standard G: 5.1 RLU 2083
Standard H: 1.69 RLU 933
Mean of negative population: RLU 401, sd. RLU **4**
150 µL of sample were used to perform the test.

A small population of negative samples coming from blood donors was also analysed.

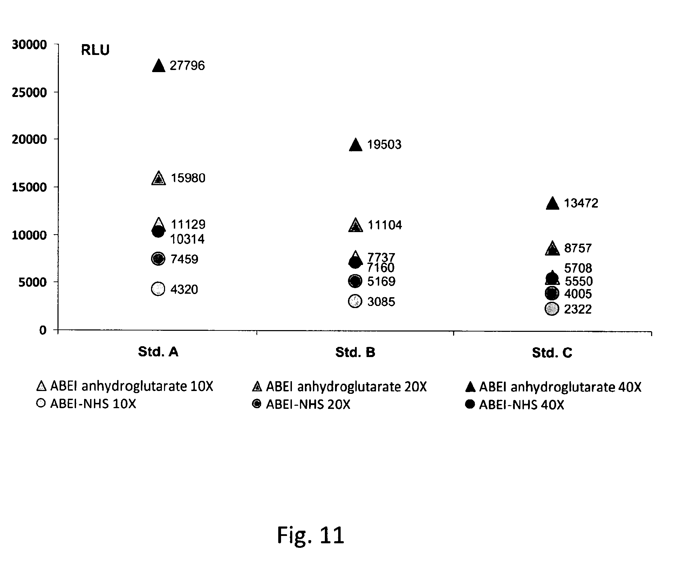

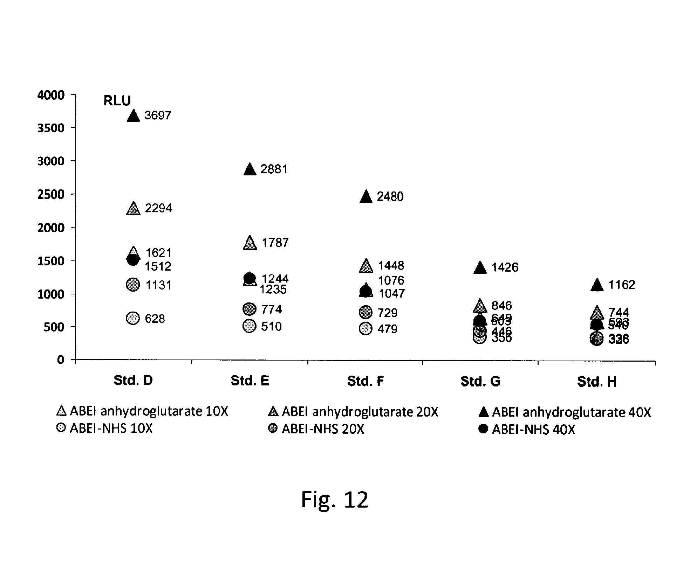

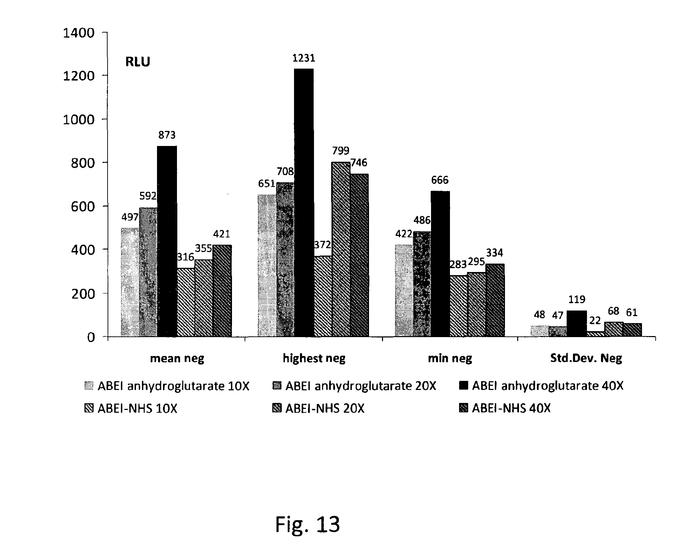

The results of the test are depicted in the figs. 11,12,13.

### Summary of Immunoassay steps:

1. sample 150 µL
2. addition of sample diluent 30 µL
3. addition of paramagnetic particles 20 µL
4. incubation at 37°C, 10 minutes
5. addition of tracer 200 µL
6. incubation at 37°C 20 minutes
7. wash
8. addition of starter reagents
9. measurement of RLU

### PRINCIPLE OF THE ASSAY n.2

The method for qualitative HBsAg determination is a direct sandwich chemiluminescence immunoassay (CLIA),

Antibody to HBsAg (mouse monoclonal antibody) was used for coating magnetic particles (solid phase) and a second monoclonal antibody to a different HBsAg epitope was conjugated to ABEI anhydroglutarate (tracer) or ABEI-NHS (tracer) using the molar ratios 10x 20x, 40x.

During the first incubation, HBsAg present in samples binds to the solid phase. During the second incubation, the antibody conjugate reacts with HBsAg already bound to the solid phase. After the second incubation, the unbound material is removed with a wash cycle. Subsequently, starter reagents are added and a flash chemiluminescence reaction is induced. The light signal, and hence the amount of ABEI anhydroglutarate-antibody conjugate, is measured by a photomultiplier as relative light units (RLU) and is indicative of HBsAg the concentration present in the samples.

Apart the two Mabs (solid phase and tracer Mabs) the reagents, the working concentration and the assay method were the same as for the current Liaison HBsAg kit (DiaSorin cat #310100).

We used as positive samples a standard curve (8 samples from A till H) at known concentration of HBsAg obtained by serial dilution in negative serum of high positive HBsAg sample (see the assay N.1).

150 µL of sample were used to perform the test.

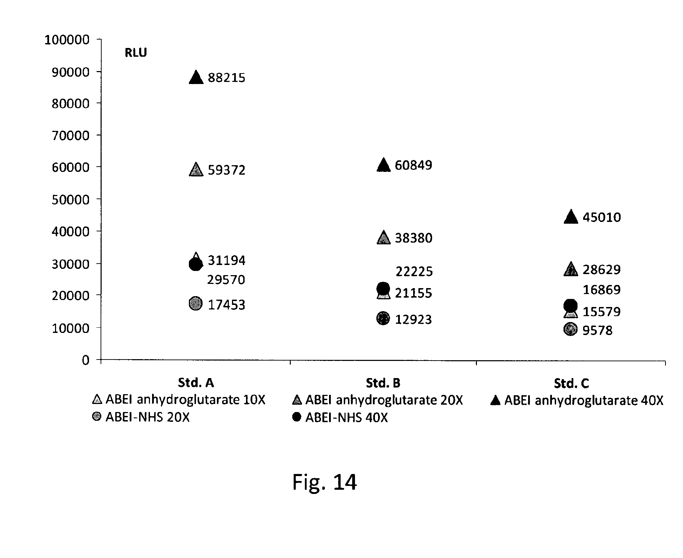

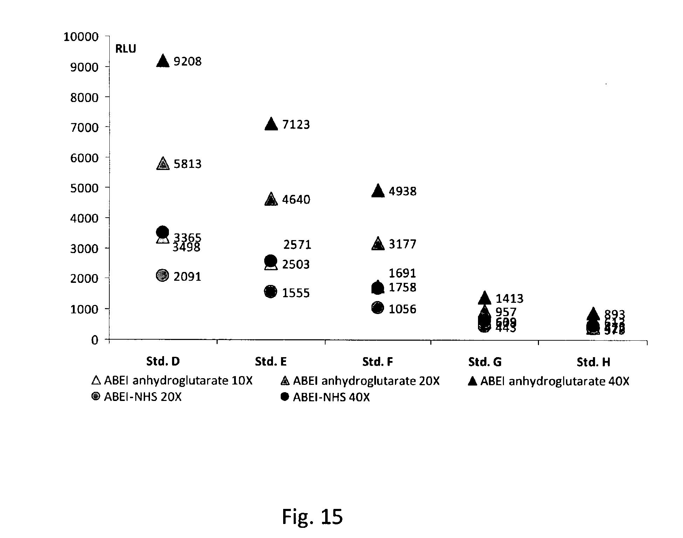

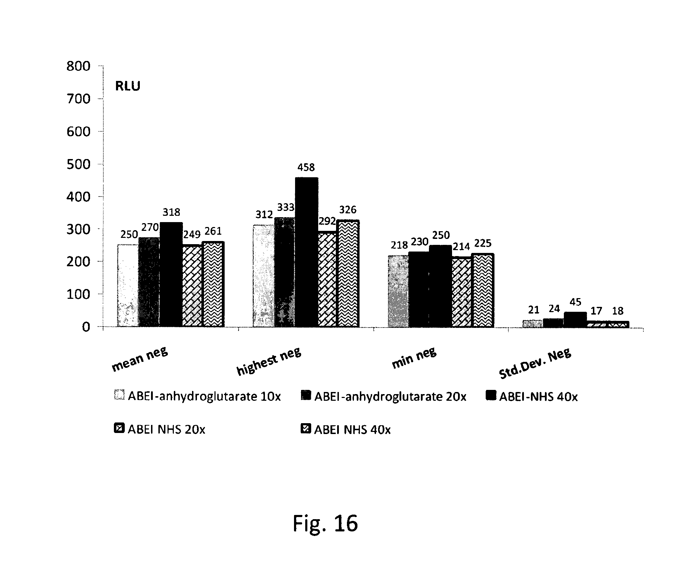

A small population of negative samples coming from blood donors was also analysed. The results of the test are depicted in the figs. 14, 15, 16.

### Summary of Immunoassay steps:

1. sample 150 µL
2. addition of sample diluent 30 µL
3. addition of paramagnetic particles 20 µL
4. incubation at 37°C, 10 minutes
5. addition of tracer 200 µL
6. incubation at 37°C 20 minutes
7. wash
8. addition of starter reagents
9. measurement of RLU

### PRINCIPLE OF THE ASSAY n.3

While the previous two model systems illustrate the conjugation of ABEI anhydroglutarate to antibody molecules many of the reagents that are used in in vitro diagnostic assays are recombinant protein molecules. The study describes an assay that was used to develop a highly sensitive chemiluminescence method for qualitative determination of IgG against HIV gp41 antigen in a sandwich chemiluminescence immunoassay (CLIA).

In this immunoassay format the antigen sandwich method was employed in which the chemiluminescent molecule is conjugated to the recombinant HIV gp41 antigen (similar to that also immobilized on the solid phase). The antibody in the sample is "sandwiched" between 2 antigen molecules, one immobilized on the solid phase and one containing the label (ABEI anhydroglutarate -antigen conjugate). Subsequently, the unbound material is removed with a wash step and the addition of the starter solutions results in the development of a light signal in proportion to the antibody concentration. The light signal, and hence the amount of ABEI anhydroglutarate-antibody conjugate, is measured by a photomultiplier as relative light units (RLU) and is indicative of the IgG anti-HIV concentration present in samples.

We used as positive samples several diluted sera obtained by serial dilution in HIV negative serum of two (n. 502 and n. 504) high positive HIV sample. 100 µL of sample were used to perform the test. A small population of negative samples coming from an open population was also analysed.

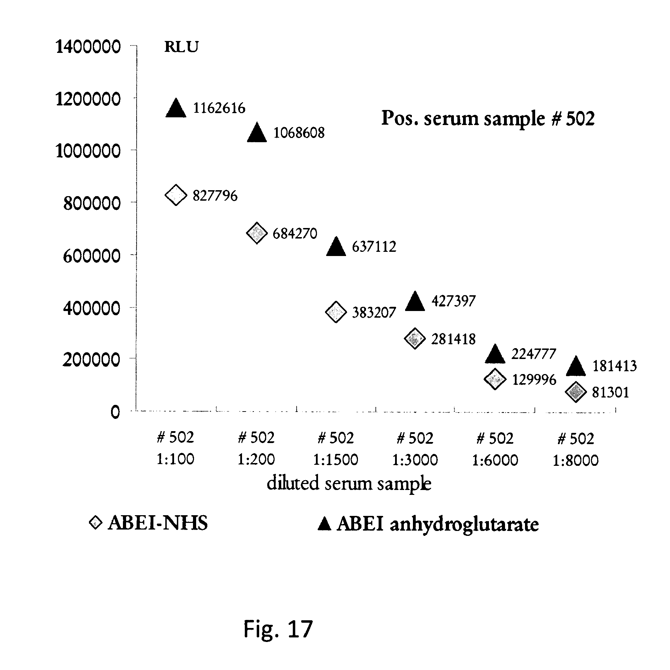

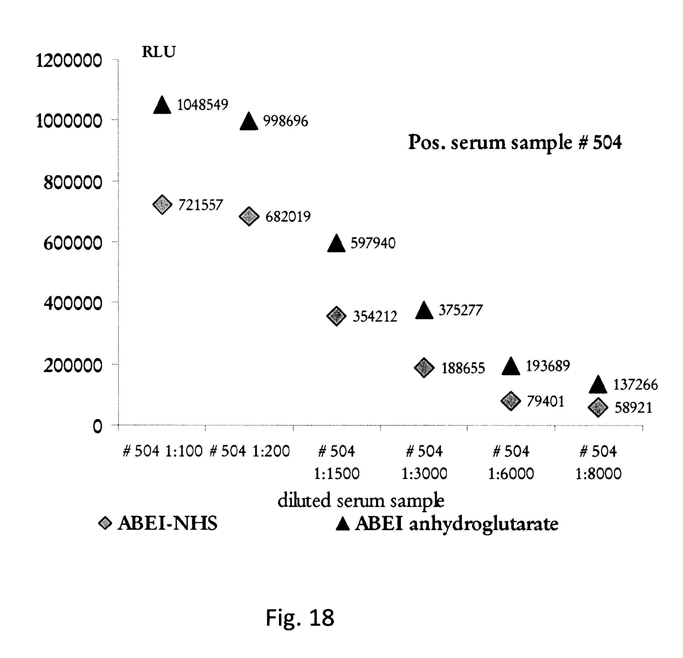

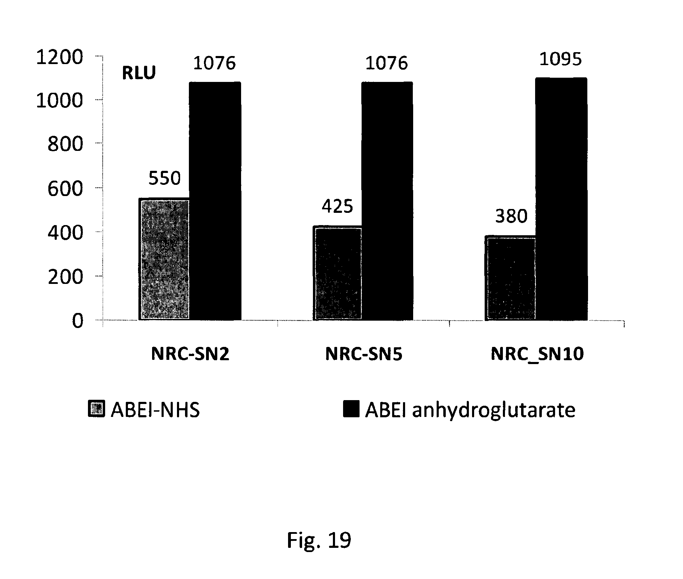

The results of the test are depicted in the figs. 17, 18, 19.

We also evaluated the stability after thermal stress of the tracer (ABEI anhydroglutarate-antigen conjugate); the tracer was diluted at 0.5 µg/ml and subsequently incubated at 37° C for 3 and 6 days and at 45°C for 3 days; after 3 and 6 days the tracer was removed from the incubator and stored at 4°C. On the seventh day all the immunoassay measures were run at the same time with the same instrument. We used as reference the RLU obtained from the tracer stored at 4°C.

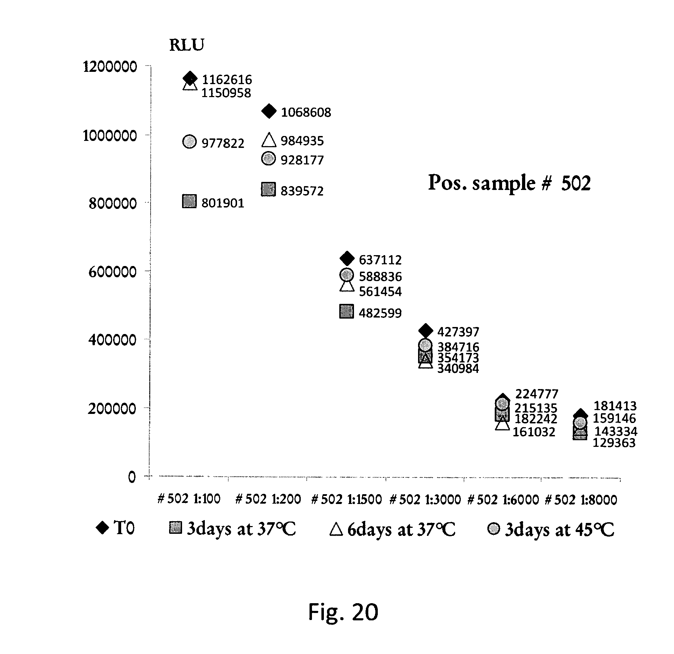

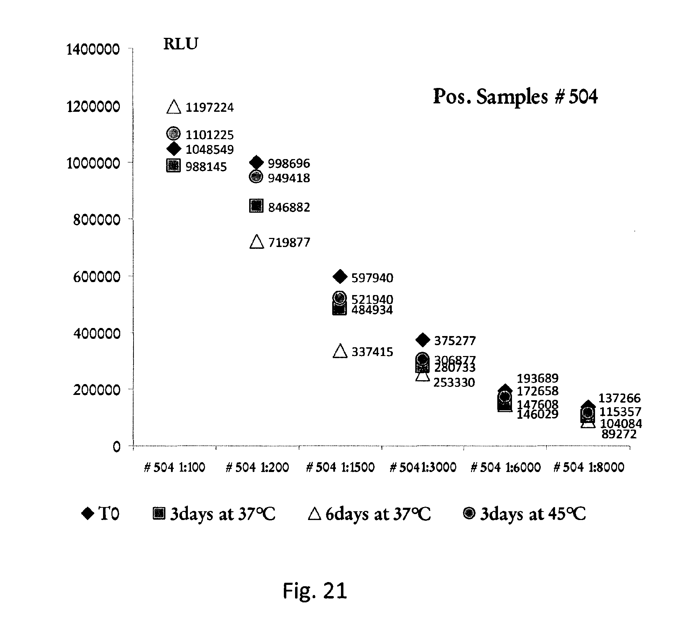

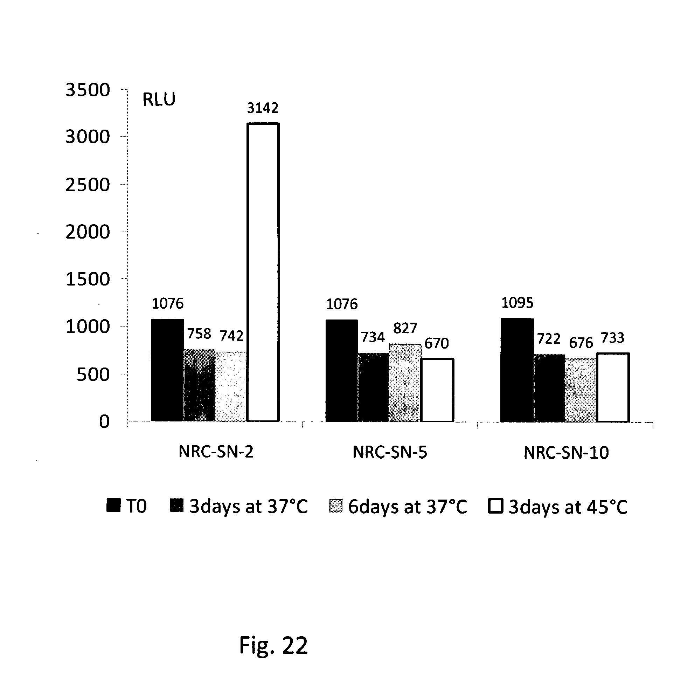

The results of the test are depicted in the figs. 20, 21, 22.

### Immunoassay steps:

1. sample 100 µL
2. addition of sample diluent 40 µL
3. addition of paramagnetic particles 10 µL
4. incubation at 37°C 10 minutes
5. addition of tracer diluent 180 10 µL
6. addition of tracer 20 µL
7. incubation at 37°C 30 minutes
8. wash
9. addition of starter reagents
10. measurement of RLU

## Claims

1. Compound of general formula (1) 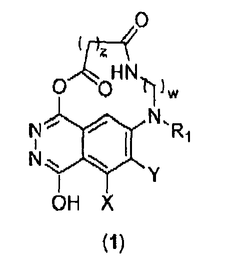 wherein:
X and Y, independently from one another, represent hydrogen, halide (Cl, Br, F, I), hydroxyl, primary or secondary amine, thiol, metboxy, alkyloxy or alkyl group;
- z is an integer number between 1 and 6;
- w is an integer number between 2 and 6;
- R₁ is C1-C5 alkyl.

2. The compound according to claim 1 wherein X and Y are hydrogen, z = 2 or 3, w = 4 and R₁ = C2 alkyl (ethyl).

3. The compound according to claim 2 being *N*-(4-Aminobutyl)-*N*-ethylisoluminol anhydroglutarate (ABEI anhydroglutarate).

4. A chemiluminescent conjugate consisting of a first molecule containing a nucleophilic group and a second molecule being the compound according to claims 1 to 3, said second molecule being covalently attached to the nucleophilic group of said first molecule.

5. The chemiluminescent conjugate according to claim 4 wherein said first molecule is selected among those containing a primary amine, a secondary amine, a thiol or an alcohol.

6. The chemiluminescent conjugate according to claim 5 wherein the first molecule is a molecule containing at least one lysine residue and the second molecule is covalently attached to the free primary amine of said lysine residue.

7. The chemiluminescent conjugate according to claim 6 wherein the lysine residue containing molecule is a protein, a recombinant protein, a peptide or a synthetic peptide derivative.

8. The chemiluminescent conjugate according to claim 7 wherein the lysine residue containing protein is an immunoglobulin or a synthetic or recombinant derivative thereof.

9. The chemiluminescent conjugate according to claim 8 wherein the immunoglobulin or a synthetic or recombinant derivative thereof is able to specifically bind to a pathogen derived antigen.

10. The chemiluminescent conjugate according to claim 9 wherein the immunoglobulin or a synthetic or recombinant derivative thereof is able to specifically bind to a HBV antigen.

11. The chemiluminescent conjugate according to claim 10 wherein the immunoglobulin or a synthetic or recombinant derivative thereof is able to specifically bind to the HBsAg.

12. The chemiluminescent conjugate according to claim 9 wherein the immunoglobulin or a synthetic or recombinant derivative thereof is able to specifically bind to a HCV antigen.

13. The chemiluminescent conjugate according to claim 9 wherein the immunoglobulin or a synthetic or recombinant derivative thereof is able to specifically bind to a HIV antigen.

14. The chemiluminescent conjugate according to claim 9 wherein the immunoglobulin or a synthetic or recombinant derivative thereof is able to specifically bind to HIV gp41.

15. Use of the compound according to claims 1 to 3 to label a molecule containing a nucleophilic group and obtaining the chemiluminescent conjugate of any of claims 4 to 14.

16. Use of the compound according to claims 1 to 3 to label a molecule containing at least one lysine residue and obtaining the chemiluminescent conjugate of any of claims 6 to 14.

17. An immunoassay for the detection of an antigen that makes use of the chemiluminescent conjugate according to any of claims 4 to 14 as labeled probe.

18. An immunodiagnostic kit comprising the chemiluminescent conjugate according to any of claims 4 to 14 as labeled probe.
